# EUROPEAN PATENT APPLICATION

(11) **EP 2 548 593 A1**
(43) Date of publication of application: **23.01.2013**
(21) Application number: 11755969.0
(22) Date of filing: 28.01.2011
(51) Int. Cl.: A61M 1/10, A61M 1/12

(54) **MEDICAL INFLATION/DEFLATION DRIVE DEVICE**

(30) Priority: 23.03.2010 JP 2010065513; 17.03.2010 JP 2010060054
(71) Applicant: Zeon Corporation, Tokyo 100-8246 (JP)
(72) Inventor: KUROKI, Hidehiro, Tokyo 100-8246 (JP); HASHIOKA, Shingi, Tokyo 100-8246 (JP); ASANO, Junichi, Tokyo 100-8246 (JP)
(74) Representative: Vossius & Partner
(86) International application number: PCT/JP2011/051688
(87) International publication number: WO 2011/114779

(57) **Abstract**

A medical inflation/deflation drive device comprises: pressure generation means (11, 12, 40) which, in order to repeat the inflation and deflation of a driven device (22), alternately applies positive pressure and negative pressure to a piping system (18) communicating with the driven device (22); a gas tank (71) which, according to the difference between the pressure in the piping system (18) and the pressure in the gas tank (71), sucks a part of gas within the piping system (18) or discharges a part of the gas within the gas tank (71) to the piping system (18); a valve means (72) for selectively opening and closing the connection between the gas tank (71) and the piping system (18) ; and a control means for controlling the valve means (72) in such a manner that the valve means (72) is opened after a predetermined period of time after the time point at which the application of the pressure by the pressure generation means (11, 12, 40) is switched from the application of the positive pressure or the negative pressure to the application of the negative pressure or the positive pressure, and that the valve means (72) is closed before the next switching.

## Description

### {Technical Field}

The present invention relates to a medical inflation/deflation drive device, which drives to inflate/deflate a medical device such as an intra-aortic balloon pump (IABP) by alternately outputting positive pressure and negative pressure, for example.

### {Background Art}

For example, in an IABP balloon catheter, a balloon thereof is inserted into an aortic vessel near a heart of a patient and this is inflated and deflated in accordance with a heartbeat, thereby performing cardiac assist treatment. Drive devices as disclosed in following patent literatures 1 and 2 are known as the drive device for inflating and deflating the balloon.

The drive devices disclosed in the publications including a primary side piping system and a secondary side piping system separate the systems by a pressure transmission partition device (generally referred to as a volume limiting device (VLD) or an isolator), transmit change in pressure generated in the primary side piping system to the secondary side piping system and drive to inflate and deflate the balloon by the change in pressure generated in the secondary piping system. The primary piping system and the secondary piping system are separated in this manner so as to make fluid for driving the balloon and the fluid, which generates the positive pressure and the negative pressure, different from each other for improving responsiveness of inflation and deflation of the balloon. Also, this is for inhibiting large consumption of relatively expensive fluid in the secondary piping system, that is to say, generating the pressure at a low cost by hermetically maintaining the secondary piping system by eliminating leakage by diffusion. Helium gas of which mass is low and excellent in responsiveness is preferably used as the gas enclosed in the secondary piping system.

In such medical inflation/deflation drive device, it is desired to further improve the responsiveness at the time of the inflation and the deflation of the balloon, that is to say, to shorten time required from application of the positive pressure for inflating the balloon until the balloon is fully inflated and time required from application of the negative pressure for deflating the balloon until the balloon is fully deflated so as to be able to sufficiently follow a rapid pulse of the patient. Especially, for improving handling, it is desired to respond to a longer piping (drive tube length) from the drive device to the balloon, a larger balloon volume for a relatively strongly-built patient, and the catheter of which diameter is smaller for reducing physical strain on the patient, and it is required to ensure sufficient responsiveness also in such cases.

Also, the gas enclosed in the secondary piping system permeates a balloon membrane and a wall of the tube, which composes the piping system, to diffuse over time, and this might be contaminated by invasion of a blood component, so that it is required to entirely change the gas in the secondary piping system at regular intervals (full purge). The full purge is conventionally performed by connecting the secondary piping system to a vacuum pump to discharge all the gas therein and thereafter filling the secondary piping system with the gas from a tank in which new gas is enclosed. Therefore, this cannot be performed in a state in which the medical inflation/deflation device operates (state in which inflation/deflation operation of a driven device is continuously performed) and this should be performed in a state in which a function thereof is deactivated. This might put the strain on the patient under treatment, so that improvement thereof is desired.

The present invention is achieved in consideration of such points and a first object thereof is to provide the medical inflation/deflation drive device excellent in responsiveness.

A second object thereof is to provide the medical inflation/deflation drive device capable of changing the gas without stopping the inflation and the deflation of the driven device.

### {Citation List}

### {Patent Literature}

Patent Literature 1: Japanese Patent Publication No. 3767008
Patent Literature 2: Japanese Patent Publication No. 3804092

### {Summary of Invention}

### {Solution to Problem}

(1) A medical inflation/deflation drive device according to a first aspect of the present invention includes: pressure generation means, which, in order to repeat inflation and deflation of a driven device, alternatively applies positive pressure and negative pressure to a piping system communicating with the driven device; a gas tank, which sucks a part of gas in the piping system or discharges a part of the gas in the gas tank to the piping system according to difference between pressure in the piping system and the pressure in the gas tank; valve means, which selectively opens and closes communication between the gas tank and the piping system; and control means, which controls in such a manner that the valve means is opened after a predetermined period of time after a point of time at which application of the pressure by the pressure generation means is switched from the application of the positive pressure or the negative pressure to the application of the negative pressure or the positive pressure, and that the valve means is closed before next switching.

In the present invention, the IABP balloon catheter may be preferably illustrated, for example, as the driven device. Although not especially limited, the pressure generation means preferably includes primary side pressure generation means, which alternately generates the positive pressure and the negative pressure, and secondary side pressure generation means composed of pressure transmission partition means in which a first chamber into which the positive pressure and the negative pressure generated by the primary side pressure generation means are alternately introduced through the primary piping system and a second chamber hermetically separated from the first chamber to which at least a part of the pressure in the first chamber is transmitted, are formed.

The change in pressure in the piping system associated with the application of the positive pressure or the negative pressure by the pressure generation means is such that, when the positive pressure is applied to the piping system, the pressure in the piping system increases to overshoot plateau pressure (pressure when the driven device is fully inflated), then decreases to the plateau pressure to be maintained substantially constant until next switching (switching to the negative pressure). Thereafter, by the switching to the negative pressure, the pressure in the piping system decreases to overshoot reference pressure (pressure when the driven device is fully deflated), then increases to the reference pressure to be maintained substantially constant until the next switching (switching to the positive pressure), and they are sequentially repeated. In the present invention, the valve means is opened after the predetermined period of time after the time point at which it is switched to the positive pressure, and since the positive pressure is applied to the piping system at that time, a part of the gas in the piping system is sucked into the gas tank according to the difference between the pressure in the piping system and the pressure in the gas tank. On the other hand, the valve means is opened after the predetermined period of time after the time point at which it is switched to the negative pressure, and since the negative pressure is applied to the piping system at that time, a part of the gas in the gas tank is discharged to the piping system according to the difference between the pressure in the gas tank and the pressure in the piping system. It is possible to decrease difference between the plateau pressure and the reference pressure by optimizing the predetermined period of time when the positive pressure is applied and the predetermined period of time when the negative pressure is applied (for example, this is set to a time point at which the driven device is fully inflated or fully deflated), therefore, time taken to reach the plateau pressure from the reference pressure may be made shorter at the time of the inflation and the time taken to reach the reference pressure from the plateau pressure may be made shorter at the time of the deflation, so that the responsiveness related to the inflation and the deflation of the driven device may be improved.

(2) A medical inflation/deflation drive device according to a second aspect of the present invention includes: pressure generation means, which, in order to repeat inflation and deflation of a driven device, alternately applies positive pressure and negative pressure to a piping system communicating with the driven device; a gas tank, which sucks a part of gas in the piping system or discharges a part of the gas in the gas tank to the piping system according to difference between pressure in the piping system and the pressure in the gas tank; valve means, which selectively opens and closes communication between the gas tank and the piping system; gas replenishment means, which replenishes the piping system with the gas through the gas tank; gas discharge means, which discharges the gas in the piping system through the gas tank; and control means, which controls in such a manner that the valve means is opened after application of the pressure by the pressure generation means is switched from the application of the positive pressure or the negative pressure to the application of the negative pressure or the positive pressure and that the valve means is closed before next switching, and, in a state in which the valve means is closed, controls the gas discharge means to discharge at least a part of the gas in the gas tank and controls the gas replenishment means to replenish the gas tank with the gas.

In the present invention, the IABP balloon catheter may be preferably illustrated, for example, as the driven device. Although not especially limited, the pressure generation means preferably includes the primary side pressure generation means, which alternately generates the positive pressure and the negative pressure, and the secondary side pressure generation means composed of the pressure transmission partition means in which the first chamber into which the positive pressure and the negative pressure generated by the primary side pressure generation means are alternately introduced through the primary piping system and the second chamber hermetically separated from the first chamber to which at least a part of the pressure in the first chamber is transmitted, are formed.

According to the present invention, when it is required to entirely change the gas in the piping system, it is possible to change a part of the gas in the piping system (half purge) by discharging the gas from the gas tank and replenishing the gas tank with the gas in a state in which the valve means is closed, and it is possible to entirely change the gas in the piping system (full purge) by repeating this. Therefore, it becomes possible to change the gas in the piping system without stopping the inflation and the deflation of the driven device, thereby easing the strain on the patient under treatment. Also, it is possible to discharge a part of the gas in the piping system or replenish the piping system with the new gas as needed, so that it is possible to maintain gas concentration in the piping system constant, thereby maintaining an inflation rate and a deflation rate of the driven device constant.

(3) The medical inflation/deflation drive device according to a third aspect of the present invention is the medical inflation/deflation drive device according to the second aspect of the present invention, further including: piping system pressure detection means, which detects the pressure in the piping system, wherein the gas replenishment means includes: a primary gas tank in which the gas with which the gas tank is replenished is enclosed; first valve means, which may be opened and closed, connected to an output side of the primary gas tank; a secondary gas tank, which communicates with the output side of the primary gas tank by opening and closing of the first valve means; tank pressure detection means, which detects the pressure in the secondary gas tank; and second valve means connected to an output side of the secondary gas tank, which controls replenishment of the gas tank with the gas from the secondary gas tank by the opening and closing of the valve, and wherein when the pressure detected by the piping system pressure detection means at timing at which the driven device is switched from a deflated state to an inflated state becomes a predetermined value or lower, the control means closes the first valve means and opens the second valve means before opening the valve means to replenish the gas tank with the gas from the secondary gas tank and calculates a replenished amount of the gas to the piping system based on change in pressure in the secondary gas tank detected by the tank pressure detection means before and after the replenishment of the gas.

In the present invention, it is preferable that the piping system pressure detection means detects the pressure in the piping system at the timing at which the driven device is switched from the deflated state to the inflated state and the control means judges whether the detected pressure is the predetermined value or lower.

The replenished amount of the gas to the piping system on a driven device side is calculated in a following manner by using the medical inflation/deflation drive device according to the present invention. When the pressure detected by the piping system pressure detection means becomes the predetermined value or lower, the first valve means is closed and the second valve means is opened. According to this, the gas tank may be replenished with the gas from the secondary gas tank. Before and after the gas replenishment, the tank pressure detection means detects the pressure in the tank. Since the secondary gas tank is blocked from the primary gas tank by the first valve means during the same, the replenished amount of the gas moved from the secondary tank to the piping system is obtained from difference in pressure before and after the gas replenishment (P1-P2) and a volume V of the secondary gas tank. The replenished amount is proportional to (P1-P2) × V.

The replenished amount of the gas obtained in this manner is recorded over time. It is preferable to record on a semiconductor memory, a magnetic disk, an optical recording medium, and other recording media as recording means so as to be able to output on a screen or paper as needed. Then, while observing change over time in recorded replenished amount of the gas, it is possible to judge that abnormal leakage of the gas occurs when an interval between each gas replenishment starts becoming shorter and a calculated replenished amount of the gas starts increasing, thereby issuing an alert early. A central processing unit (CPU) and the like appropriately reads the change over time in the replenished amount of the gas recorded on the recording means, and when this judges that the replenished amount of the gas starts increasing, this may automatically issue the alert.

In the present invention, it becomes possible to correctly comprehend the replenished amount of the gas to the piping system on the driven device side regardless of a cause on a side of the patient and mechanical variation, to clearly distinguish the abnormal leakage by a pinhole and the like formed on the driven device or the piping system from the leakage of normal time, and to issue the alert early when there is abnormality.

(4) The medical inflation/deflation drive device according to a fourth aspect of the present invention is the medical inflation/deflation drive device according to the second aspect of the present invention, further including: deflation time calculation means, which calculates time during which the driven device is deflated; inflation stop means, which stops the inflation of the driven device one or more times continuously when deflation time calculated by the deflation time calculation means is predetermined time or shorter to make the deflation time predetermined time or longer; and pressure detection means capable of detecting the pressure in the piping system at timing just before it is switched to next inflation after the inflation is stopped one or more times by the inflation stop means, and it is configured such that the control means controls the gas replenishment means, thereby replenishing the piping system with the gas through the gas tank such that the pressure detected by the pressure detection means becomes a predetermined value.

It is possible to calculate the time during which the driven device is deflated or inflated by the deflation or inflation time calculation means by monitoring switching timing of the positive pressure and the negative pressure by the pressure generation means, for example. The inflation and the deflation of the driven device is switched in synchronization with change in blood pressure or the heartbeat of the patient, so that it is also possible to calculate the time during which the driven device is deflated/inflated based on an output signal from means for detecting the change in blood pressure or the heartbeat of the patient.

Meanwhile, in the present invention, the deflation or inflation time calculation means is intended to mean the means for calculating the time during which the driven device is deflated and/or inflated. Also, in the present invention, the deflation or inflation time is intended to mean a period during which the driven device is deflated and/or inflated.

In the medical inflation/deflation drive device according to the fourth aspect of the present invention, when the period during which the driven device is deflated or inflated calculated by the deflation or inflation time calculation means is shorter than the predetermined time, drive to inflate or deflate the driven device is stopped for one or a few beats by inflation or deflation stop means. Although the predetermined time, which is a reference in this case, is not especially limited, this is preferably set to 100 to 500 milliseconds and is more preferably set to 150 to 300 milliseconds. When the driven device is repeatedly inflated or deflated with such a period not longer than the predetermined time, it is not possible to detect the pressure in a stable deflated state or inflated state even when the pressure in the piping system is detected at the timing just before the driven device is switched from the deflated state to the inflated state or from the inflated state to the deflated state. Pressure lower than the pressure in the stable deflated state or pressure higher than the pressure in the stable inflated state at the time of normal operation of the driven device is detected.

Therefore, in the present invention, the drive to inflate or deflate the driven deice is temporarily stopped in such a case. Therefore, at timing just before it is switched to next inflation or deflation, the inner pressure of the piping system, which communicates with the driven device, is stable pressure in the deflated state or the inflated state of the driven device. In the present invention, the pressure is detected by the pressure detection means. Next, it is judged whether gas pressure is normal based on the detected pressure. When the detected pressure is lower than a predetermined threshold (for example, 0 mmHg in the deflated state: gauge pressure, and 120 mmHg in the inflated state: gauge pressure), it is considered that the gas in the piping system is really deficient, so that the piping system is replenished with the gas through the gas tank in this case. A method of replenishing the gas is not especially limited and it is possible to replenish in a short time several times or replenish a constant amount at one time.

In this manner, in the medical inflation/deflation drive device according to the fourth aspect of the present invention, it is possible to replenish the piping system including the driven device with an appropriate amount of gas even when an interval between each inflation and deflation of the driven device is short. As a result, in the present invention, various problems caused by too much gas injected into the piping system may be resolved even when the patient has a rapid heart rate. Also, at that time, although the inflation or the deflation of the driven device is stopped for one or a few beats in the present invention, a method of maintaining the deflated state is more preferable than a method of maintaining the inflated state especially for easing the strain on the heart of the patient. However, since this is only for a short time, there is little effect on the treatment by the driven device. Also, as compared to the drive device, which replaces the gas in the entire piping system at regular intervals (full purge), the present invention is economic with a small amount of gas consumption.

(5) The medical inflation/deflation drive device according to a fifth aspect of the present invention is the medical inflation/deflation drive device according to the second aspect of the present invention, further including: inflation time calculation means, which calculates time during which the driven device is inflated; inflation continuation means, which, when inflation time calculated by the inflation time calculation means is predetermined time or shorter, continues the inflation of the driven device for the predetermined time or longer; and pressure detection means capable of detecting the pressure in the piping system at timing just before it is switched to next deflation after the inflation continuation means continues the inflation for the predetermined time or longer, and it is configured such that the control means controls the gas replenishment means, thereby replenishing the piping system with the gas such that the pressure detected by the pressure detection means becomes a predetermined value.

When inclination of the change in pressure is calculated by pressure change calculation means, it is possible to store temporal differentiation of the pressure detected by the pressure detection means on the memory and the like and calculate based on the stored data.

In the medical inflation/deflation drive device according to the fifth aspect of the present invention, when the inclination of the change in pressure calculated by the pressure change calculation means is larger than a predetermined value, gas replenishment stop means stops gas replenishment operation to the piping system for a period of predetermined beats. A case in which the inclination of the change in pressure calculated by the pressure change calculation means is larger than the predetermined value is considered to be a case in which a cycle of the inflation/deflation of the driven device is short and it is switched from the deflation (or inflation) to the inflation (or deflation) before the pressure in the piping system is stabilized, for example. Since it is not possible to detect the stable pressure in the deflated state (or inflated state) correctly even when normal gas replenishment operation is performed in such a case, there is a risk that too much gas is injected into the piping system.

Therefore, in the present invention, the gas replenishment stop means stops the gas replenishment operation to the piping system in such a case. Thereafter, when the inclination of the change in pressure becomes equal to or lower than the predetermined value, the pressure detection means detects the pressure in the piping system at the timing just before the driven device is switched from the deflated state (or inflated state) to the inflated state (deflated state). When the inclination of the change in pressure is equal to or lower than the predetermined value, it is considered that the interval between each inflation/deflation of the driven device is close to that of the normal time and it is possible to detect the stable pressure in the deflated state of the driven device.

Therefore, in the present invention, it is judged whether the gas pressure is normal based on the detected pressure. When the detected pressure is lower than the predetermined threshold (for example, 0 mmHg: gauge pressure), it is considered that the gas in the piping system is really deficient, so that the piping system is replenished with the gas in such a case. The method of replenishing the gas is not especially limited and it is possible to replenish in a short time several times or replenish a constant amount at one time.

In this manner, in the medical inflation/deflation drive device according to the fifth aspect of the present invention, the pressure in the piping system is detected and the gas replenishment operation is performed based on the pressure when the inclination of the change in pressure calculated by the pressure change calculation means is equal to or lower than the predetermined valve and is stable.

This method is excellent in that this may be used also in a case in which entrance and exit of driver gas is slower than usual by variation in catheter and distortion and bend of a catheter portion in the body. In the conventional technology, it is highly possible that too much driver gas is injected in such a case.

Therefore, in the present invention, it is possible to replenish the piping system including the driven device with an appropriate amount of gas. As a result, in the present invention, too much gas is not injected into the piping system even when the heart rate of the patient becomes rapid, so that the various problems caused by this may be resolved. Also, in the present invention, since the inflation/deflation of the driven device is not basically stopped at that time, there is no effect on the treatment by the driven device. Also, as compared to the drive device, which replaces the gas in the entire piping system at regular intervals, the present invention is economic with the small amount of gas consumption.

Meanwhile, in the present invention, the timing just before the switching is intended to mean a point of time including 0 and in the vicinity of 0 (any point of time from 0 to tens of milliseconds before) when switching time is set to 0, and this is any point of time from 50 milliseconds before the switching of an electric signal to 50 milliseconds after the switching in consideration of mechanical response lag time (few milliseconds to tens of milliseconds in general) of the electric signal for switching the pressure.

(6) The medical inflation/deflation drive device according to a sixth aspect of the present invention is the medical inflation/deflation drive device according to the second aspect of the present invention, further including: pressure detection means, which detects inner pressure of the piping system; pressure change calculation means, which calculates inclination of change in pressure of the inner pressure of the piping system by the pressure detection means at timing just before the driven device is switched from a deflated state or an inflated state to the inflated state or the deflated state; and gas replenishment stop means, which stops gas replenishment operation to the piping system when an absolute value of the inclination of the change in pressure calculated by the pressure change calculation means is larger than a predetermined value, and it is configured such that, when the absolute value of the inclination of the change in pressure calculated by the pressure change calculation means is equivalent to or smaller than the predetermined value, the control means controls the gas replenishment means, thereby replenishing the piping system with the gas through the gas tank such that the inner pressure of the piping system detected by the pressure detection means becomes a predetermined value at the timing just before the driven device is switched from the deflated state or the inflated state to the inflated state or the deflated state.

The medical inflation/deflation drive device according to the sixth aspect of the present invention detects the pressure by the pressure detection means according to the fourth and fifth aspects, and there is a problem of when to stop the inflation or the deflation for one or more beats based on this if necessary. That is to say, it is preferable not to stop the inflation or the deflation even if this is only for one beat, and if the inflation or the deflation is frequently stopped for the patient of which inflation or deflation period is short, this causes frequent stop of assistance. Therefore, especially from a viewpoint of replenishing a loss by the diffusion of the gas, although detection for confirming this is not especially limited, it is sufficient to perform this at intervals of one to tens of minutes, and more preferably once per every three to ten minutes. Of course, it is desirable to monitor rapid change in pressure for every beat in addition to this.

Also, fluctuation of the heartbeat in which sufficiently long inflation or deflation may be taken is often included in a sufficiently long period of three to ten minutes. Therefore, when such sufficiently long period of the deflation or inflation or a state in which the absolute value of the inclination of the pressure is low may be obtained in a predetermined period, it is further preferable to judge necessity of the gas replenishment by using the detected pressure at that time.

In the present invention, it is possible to compose the medical inflation/deflation drive device by combining a plurality of functions of the medical inflation/deflation drive device according to the above-described fourth to sixth aspects. Also, the medical inflation/deflation drive device according to the above-described fourth to sixth aspects may further include gas replenishment means, which replenishes the piping system with the gas through the gas tank such that, if a state in which a predetermined condition is satisfied appears even once, the pressure detected by the pressure detection means at that time becomes the predetermined value while observing the deflation or inflation period and the absolute value of the inclination of the change in pressure during a predetermined period (number of heartbeats or time) before starting operation to stop the next inflation or deflation one or more times when the deflation or inflation time and the absolute value of the inclination of the change in pressure do not satisfy the predetermined condition.

Although the above-described function of the medical inflation/deflation drive device according to the present invention may be continuously operated during the operation of the drive device, this may also be activated for each predetermined period. Also, in the present invention, the piping system includes not only a flexible tube such as a tube and a hose but also a non-flexible tube, and also includes the device such as the tank connected to the tube.

### {Advantageous Effects of Invention}

According to the medical inflation/deflation drive device according to the first aspect of the present invention, it is possible to improve the responsiveness at the time of the inflation and the deflation of the driven device, that is to say, to shorten time required from the application of the positive pressure for inflating the balloon until the driven device is fully inflated and time required from the application of the negative pressure for deflating the driven device until the driven device is fully deflated. Therefore, a length of the piping from the drive device to the balloon (drive tube length) may be increased, so that it is possible to improve the handling, respond to the driven device of which volume is large, and to decrease the catheter diameter.

According to the medical inflation/deflation drive device according to the second aspect of the present invention, it becomes possible to maintain the gas concentration in the piping system constant and entirely change the gas in the piping system without stopping the drive of the driven device.

According to the medical inflation/deflation drive device according to the third aspect of the present invention, in addition to the effect of the medical inflation/deflation drive device according to the above-described second aspect of the present invention, it becomes possible to correctly comprehend the replenished amount of the gas to the piping system on the driven device side, so that it is possible to clearly distinguish the abnormal leakage caused by the pinhole and the like formed on the driven device or the piping system from the leakage of the normal time, thereby issuing the alert early when there is the abnormality.

According to the medical inflation/deflation drive device according to the fourth to sixth aspects of the present invention, in addition to the effect of the medical inflation/deflation drive device according to the above-described second aspect of the present invention, the piping system including the driven device is not filled with too much gas even when the interval between each inflation/deflation of the driven device is short or irregular. Also, the gas in the piping system does not become deficient. Therefore, an excellent treatment effect by the driven device is expected. Also, the drive of the driven device is not basically stopped in the present invention. Even when this is stopped, this is stopped only for one or few beats, so that there is no effect on the treatment by the driven device. Also, as compared to the drive device, which replaces the gas in the entire piping system at regular intervals, the present invention is economic with the small amount of gas consumption.

### {Brief Description of Drawings}

FIG. 1 is a schematic cross-sectional view illustrating an example of a balloon catheter of a first embodiment of the present invention.
FIG. 2 is a schematic diagram illustrating a usage example of the balloon catheter of the first embodiment of the present invention.
FIG. 3 is a schematic configuration diagram of a medical inflation/deflation drive device of the first embodiment of the present invention.
FIG. 4 is a cross-sectional view of a substantial part illustrating an example of a pressure transmission partition device of the first embodiment of the present invention.
FIG. 5 is a configuration diagram of a substantial part of a supplemental device of the first embodiment of the present invention.
FIG. 6 is a configuration diagram of the substantial part of the supplemental device of the first embodiment of the present invention at the time of positive pressure application.
FIG. 7 is a configuration diagram of a substantial part of the supplemental device of the first embodiment of the present invention at the time of negative pressure application.
FIG. 8 is a graph illustrating the change in pressure in the secondary piping system when the supplemental device of the first embodiment of the present invention is not allowed to operate.
FIG. 9 is a graph illustrating change in pressure in the secondary piping system when the supplemental device of the first embodiment of the present invention is allowed to operate.
FIG. 10 is a flowchart illustrating a control example of the medical inflation/deflation drive device of the first embodiment of the present invention.
FIG. 11 is a graph illustrating change over time in a replenished amount of helium gas of the first embodiment of the present invention.
FIG. 12 is a view illustrating timing of operation of a positive pressure valve, a negative pressure valve, and a supplemental valve of the medical inflation/deflation drive device of the first embodiment of the present invention.
FIG. 13 is a chart diagram illustrating timing of pressure detection of a second embodiment of the present invention.
FIG. 14 is a view illustrating change in pressure in a secondary piping system (balloon) when a pulse is rapid of the second embodiment of the present invention.
FIG. 15 is a flowchart illustrating a control flow of control means of the second embodiment of the present invention.
FIG. 16 is a flowchart illustrating another control flow of the control means of the second embodiment of the present invention.
FIG. 17 is a flowchart illustrating a control flow of control means of a third embodiment of the present invention.
FIG. 18 is a schematic configuration diagram of a modified example of a medical inflation/deflation drive device of the embodiment of the present invention.

### {Description of Embodiments}

A medical inflation/deflation drive device according to an embodiment of the present invention is hereinafter described in detail with reference to the drawings.

### (First Embodiment)

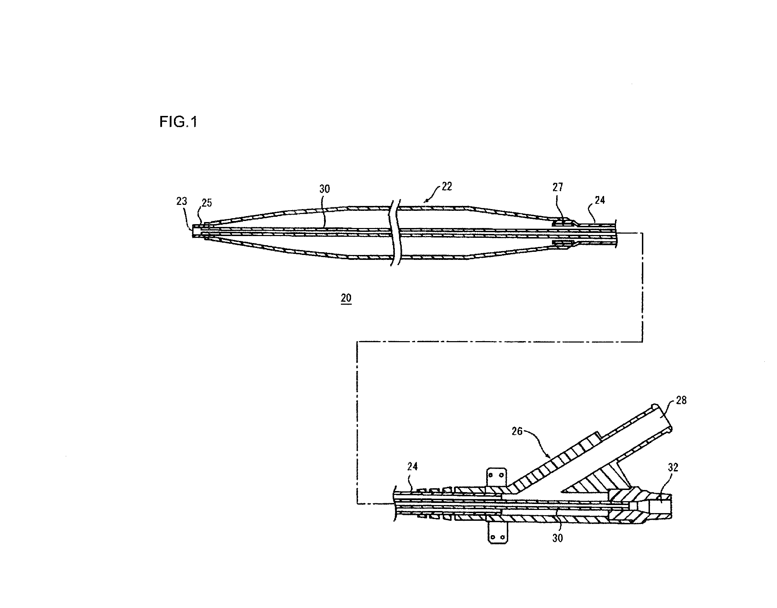

A medical inflation/deflation drive device according to this embodiment is used for inflating and deflating a balloon of an IABP balloon catheter. Before describing the medical inflation/deflation drive device according to this embodiment, an IABP balloon catheter 20 is first described with reference to FIG. 1.

As illustrated in FIG. 1, the IABP balloon catheter 20 includes a balloon 22, which is inflated and deflated in accordance with a heartbeat. The balloon 22 is composed of a cylindrical balloon membrane having a thickness of approximately 50 to 150 *µ*m. Although the balloon membrane in an inflated state has a cylindrical shape in this embodiment, the shape is not limited thereto and may be a polygonal cylindrical shape.

The IABP balloon 22 is formed of a material excellent in bending fatigue resistance. An outer diameter and a length of the balloon 22 are determined according to an inner volume of the balloon 22, which significantly affects an effect to assist a cardiac function, an inner diameter of an arterial vessel and the like. The balloon 22 has, in general, the inner volume of 30 to 50 cc, the outer diameter of 14 to 16 mm at the time of inflation, and the length of 210 to 270 mm.

A distal end of the balloon 22 is attached to an outer periphery of a distal end of an inner tube 30 directly or through a short tube 25 by means of thermal fusion bonding or adhesive bonding. A proximal end of the balloon 22 is joined to a distal end of a catheter tube 24 directly or through a contrast marker such as a metal tube 27. Pressure fluid is introduced into or removed from the balloon 22 through a first lumen formed in the catheter tube 24 to inflate or deflate the balloon 22. The balloon 22 and the catheter tube 24 are joined to each other by means of the thermal fusion bonding or the adhesive bonding with an adhesive such as an ultraviolet curable resin.

The distal end of the inner tube 30 projects farther than the distal end of the catheter tube 24. The inner tube 30 is allowed to pass through the balloon 22 and the catheter tube 24 in an axial direction. A proximal end of the inner tube 30 communicates with a second port 32 of a branching unit 26. A second lumen, which does not communicate with the inside of the balloon 22 and the first lumen formed in the catheter tube 24, is formed in the inner tube 30. The inner tube 30 sends blood pressure taken in through an opening end 23 on the distal end thereof to the second port 32 of the branching unit 26 through which change in blood pressure is measured.

When inserting the balloon catheter 20 into an artery, the second lumen of the inner tube 30 located in the balloon 22 is also used as a guide wire insertion lumen for conveniently inserting the balloon 22 into the artery. When inserting the balloon catheter into a body cavity such as a vessel, the balloon 22 is folded to be wound around the outer periphery of the inner tube 30. The inner tube 30 is formed of a material similar to that of the catheter tube 24, for example. An inner diameter of the inner tube 30 is not especially limited as far as this is the diameter with which a guide wire may pass through the inner tube 30 and this is set to, for example, 0.15 to 1.5 mm, and is preferably set to 0.5 to 1 mm. A thickness of the inner tube 30 is preferably set to 0.1 to 0.4 mm. A length of the inner tube 30 is determined according to a length of the balloon catheter 20 to be inserted into the vessel in an axial direction and the like, and although this is not especially limited, this is set to, for example, 500 to 1200 mm and is preferably set to approximately 700 to 1000 mm.

The catheter tube 24 is preferably formed of a material having a certain degree of flexibility. An inner diameter of the catheter tube 24 is preferably set to 1.5 to 4.0 mm, and a thickness of the catheter tube 24 is preferably set to 0.05 to 0.4 mm. A length of the catheter tube 24 is preferably set to approximately 300 to 800 mm.

The branching unit 26 installed outside of a patient's body is coupled to a proximal end of the catheter tube 24. The branching unit 26 is formed separate from the catheter tube 24 and is fixed thereto by means of the thermal fusion bonding, the adhesive bonding and the like. A first port 28 for introducing or removing the pressure fluid into or from the first lumen in the catheter tube 24 and the balloon 22 and the second port 32, which communicates with the second lumen of the inner tube 30, are formed on the branching unit 26.

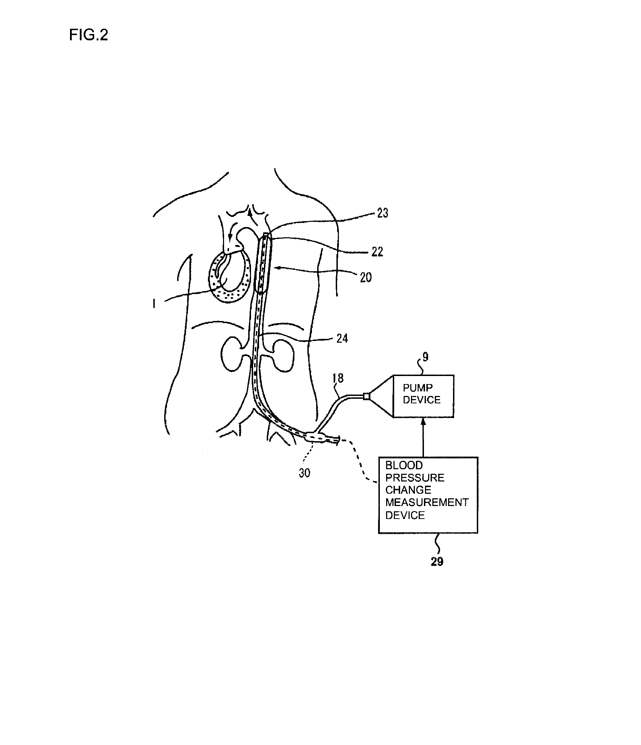

The first port 28 is connected to a pump device 9 illustrated in FIG. 2, for example, and the pump device 9 introduces or removes fluid pressure into or from the balloon 22. For quick inflation or deflation of the balloon 22 according to drive of the pump device 9, helium gas and the like with small viscosity and low mass is used as the fluid to be introduced, but is not especially limited thereto.

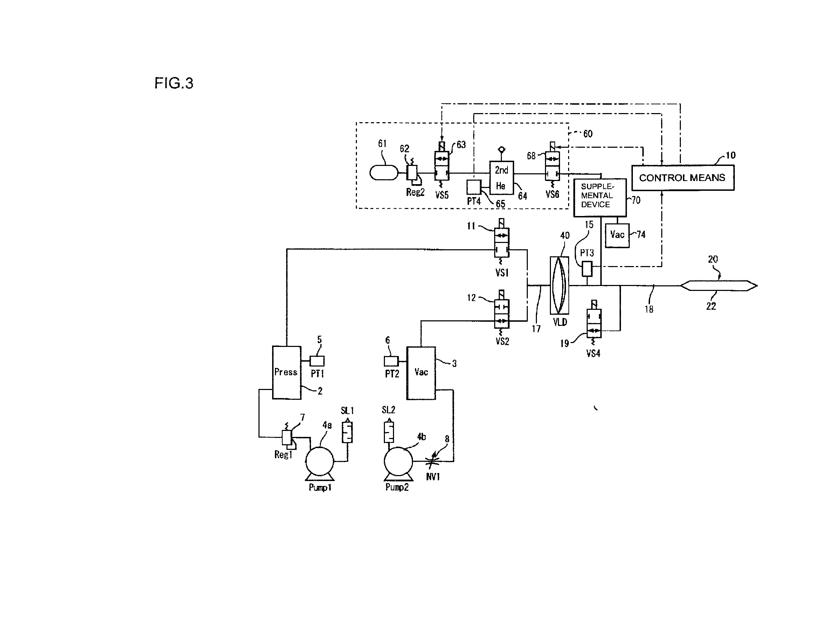

The pump device 9 will be described later in detail with reference to FIG. 3.

The second port 32 is connected to a blood pressure change measurement device 29 illustrated in FIG. 2 such that the change in blood pressure in the artery taken from the opening end 23 on the distal end of the balloon 22 may be measured. It is configured to inflate and deflate the balloon 22 with a short period of 0.4 to 1 second by controlling the pump device 9 according to the beat of a heart 1 illustrated in FIG. 2 based on the change in blood pressure measured by the blood pressure measurement device 29.

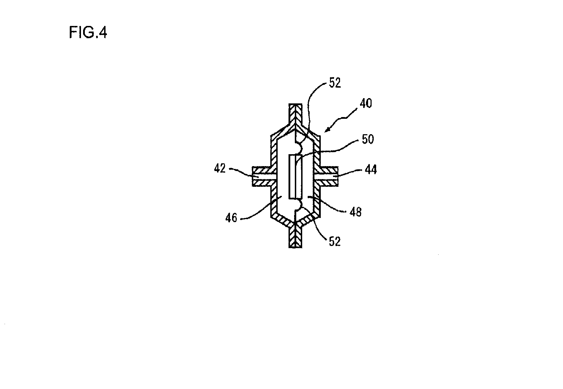

In the IABP balloon catheter 20, as described above, the helium gas and the like with the small viscosity and the low mass is used as the fluid to be introduced into and removed from the balloon 22 for more quickly inflating and deflating the balloon. Since it is not economic to generate positive pressure and negative pressure of the helium gas directly by a pump, a compressor and the like in consideration of the fact that the helium gas is lost by leakage from a seal portion and the like, a structure illustrated in FIG. 3 is adopted. That is to say, a secondary piping system 18, which communicates with the inside of the balloon 22, and a primary piping system 17, which communicates with pumps 4a and 4b as primary side pressure generation means, are separated from each other by a pressure transmission partition device 40. The pressure transmission partition device 40 includes a first chamber 46 and a second chamber 48 hermetically separated from each other by a diaphragm 52 and a plate 50 as illustrated in FIG. 4, for example.

The first chamber 46 communicates with the primary piping system 17 illustrated in FIG. 3 through a port 42. The second chamber 48 communicates with the second piping system 18 through a port 44. Although communication of the fluid is blocked between the first chamber 46 and the second chamber 48, change in pressure (change in volume) in the first chamber 46 is transmitted as the change in pressure (change in volume) in the second chamber 48 by displacement of the diaphragm 52. By adopting such a structure, it is possible to transmit the change in pressure in the primary piping system 17 to the secondary piping system 18 without allowing the primary piping system 17 and the secondary piping system 18 to communicate with each other. Also, a volume (chemical equivalent) of the gas enclosed in the secondary piping system 18 may be easily controlled constant.

In this embodiment, air is used as inner fluid of the primary piping system 17 and the helium gas is used as the inner fluid of the secondary piping system 18. The helium gas is used as the inner fluid of the secondary piping system 18 for improving responsiveness of the inflation and the deflation of the balloon 22 by using the gas with the small viscosity and the low mass.

As illustrated in FIG. 3, the two pumps 4a and 4b are arranged in the primary piping system 17 as the primary side pressure generation means. One first pump 4a is a positive pressure generating pump (also referred to as a compressor; same shall apply hereinafter) and the other second pump 4b is a negative pressure generating pump. A first pressure tank 2 as a positive pressure tank is connected to a positive pressure output port of the first pump 4a through a reduction valve 7. Also, a second pressure tank 3 as a negative pressure tank (vacuum tank) is connected to a negative pressure output port of the second pump 4b through a throttle valve 8.

Pressure sensors 5 and 6 are mounted on the first pressure tank 2 and the second pressure tank 3, respectively, as pressure detection means for detecting inner pressure thereof. The pressure tanks 2 and 3 are connected to input ports of a solenoid valve (positive pressure valve) 11 and a solenoid valve (negative pressure valve) 12, respectively. Control means 10 controls opening/closing of the solenoid valves 11 and 12 so as to correspond to the heartbeat of the patient, for example. Output ports of the solenoid valves 11 and 12 are connected to the input port 42 (refer to FIG. 4) of the pressure transmission partition device 40 as secondary side pressure generation means.

The output port 44 of the pressure transmission partition device 40 illustrated in Fig. 4 is connected to the secondary piping system 18 illustrated in FIG. 3. The secondary piping system 18 communicates with the inside of the balloon 22 and is a hermetically-closed system in which the helium gas is enclosed. The secondary piping system 18 is composed of a hose, a tube and the like. A pressure sensor 15 is mounted on the secondary piping system 18 as piping system pressure detection means for detecting inner pressure thereof. An output of the pressure sensor 15 is input to the control means 10.

Although not illustrated, an exhaust pump is connected to the secondary piping system 18 through a solenoid valve. The solenoid valve and the exhaust pump are used for evacuating the piping system 18 in order to replace the inside of the secondary piping system 18 with the helium gas before using the balloon catheter: the solenoid valve is closed and the exhaust pump is not driven in a normal use state.

Further, a solenoid valve 19 is mounted on the secondary piping system 18 and it is configured such that, when gas pressure of the secondary piping system 18 increases to a predetermined value or higher, the solenoid valve 19 opens for a predetermined period of time to let the inner gas out. The control means 10 performs this control. Further, a replenishment device 60 for replenishing the second piping system 18 with a predetermined amount of helium gas such that the chemical equivalent of the gas is always maintained constant is connected to the secondary piping system 18 through a supplemental device 70.

The replenishment device 60 includes a primary helium gas tank 61 as a primary gas tank. A first solenoid valve 63 as first valve means is connected to an output side of the helium gas tank 61 through a reduction valve 62. The control means 10 controls the opening/closing of the first solenoid valve 63. A secondary helium gas tank as a secondary gas tank is connected to an output side of the first solenoid valve 63 and a secondary helium gas tank 64 communicates with the output side of the primary helium gas tank 61 by the opening and closing of the solenoid valve 63.

A pressure sensor 65 as tank pressure detection means is mounted on the secondary helium gas tank 64 for detecting pressure in the tank 64 and it is controlled such that the pressure in the tank 64 is maintained substantially constant. For example, the pressure in the tank 64 is controlled to be approximately 100 mmHg or lower. The pressure detected by the pressure sensor 65 is input to the control means 10.

A second solenoid valve 68 as second valve means is connected to the secondary helium tank 64. The control means 10 controls the solenoid valve 68. Although not illustrated, an initial filling solenoid valve is connected in parallel to the solenoid valve 68. The initial filling solenoid valve is used when filling the secondary piping system 18 having the negative pressure with the helium gas for the first time. The solenoid valve does not operate in the normal use state.

Pressure PT1 in the first pressure tank 2 is set to approximately 300 mmHg (gauge pressure), for example, by drive of the pump 4a and pressure PT2 in the second pressure tank 3 is set to approximately -150 mmHg (gauge pressure), for example, by drive of the pump 4b. Then, the pressure applied to the input port of the pressure transmission partition device 40 illustrated in FIG. 3 is switched to the pressure of the first pressure tank 2 and that of the second pressure tank 3 by alternately driving the solenoid valves 11 and 12. The control means 10 controls switching timing in accordance with the heartbeat of the patient.

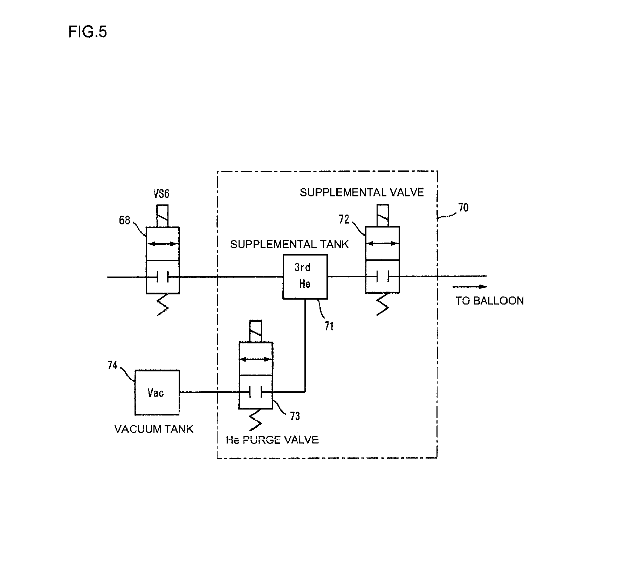

In this embodiment, the replenishment device 60 is connected to the secondary piping system 18 through the supplemental device 70. The supplemental device 70 is provided with a supplemental tank (gas tank) 71 and a supplemental valve (valve means) 72 as illustrated in FIG. 5. The supplemental tank 71 is a helium gas tank (tertiary helium tank), which sucks a part of the helium gas in the secondary piping system 18 or discharges a part of the helium gas therein to the secondary piping system 18 according to difference between the pressure in the secondary piping system 18 and the pressure therein and is connected to the secondary piping system 18 through the supplemental valve 72. The supplemental valve 72 is the solenoid valve, which selectively opens and closes the communication between the supplemental tank 71 and the secondary piping system 18 of which opening/closing operation is controlled by the control means 10 at predetermined timing.

To the supplemental tank 71, the second helium tank 64 is connected through the second solenoid valve 68 of the replenishment device 60 and a vacuum tank 74 is connected through a purge valve 73. The purge valve 73 is the solenoid valve, which selectively opens and closes the communication between the supplemental tank 71 and the vacuum tank 74 of which opening/closing operation is controlled by the control means 10. Meanwhile, it is also possible to connect the purge valve 73 to the second pressure tank 3 illustrated in FIG. 3 without providing the vacuum tank 74.

The control means 10 controls in such a manner that the solenoid valve 11 is closed after a predetermined period of time (for example, 150 msec) after a time point at which the solenoid valve (positive pressure valve) 11 is opened and application of the positive pressure to the secondary piping system 18 is started by the pressure transmission partition device 40 (time point at which it is switched from the negative pressure to the positive pressure), that the supplemental valve 72 is opened after a predetermined period of time (for example, 160 msec) after the time point at which the application of the positive pressure to the secondary piping system 18 is started (time point at which it is switched from the negative pressure to the positive pressure) (that is to say, 10 msec after the solenoid valve 11 is closed), and that the supplemental valve 72 is closed before next switching (switching from the positive pressure to the negative pressure) (for example, 10 msec before the next switching).

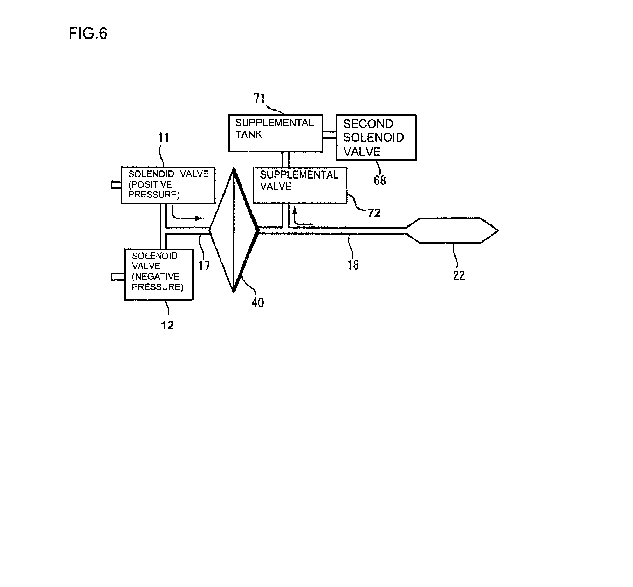

As illustrated in FIG. 6, when the supplemental valve 72 is opened in a state in which the solenoid valve 11 is opened and the positive pressure is applied to the secondary piping system 18, a part of the helium gas in the secondary piping system 18 is sucked into the supplemental tank 71 as indicated by an arrow in the drawing according to the difference between the pressure in the secondary piping system 18 and the pressure in the supplemental tank 71, and according to this, the pressure (plateau pressure) in the secondary piping system 18 decreases by an amount of the helium gas sucked by the supplemental tank 71.

Similarly, the control means 10 controls in such a manner that the solenoid valve 12 is closed after a predetermined period of time (for example, 150 msec) after a time point at which the solenoid valve (negative pressure valve) 12 is opened and application of the negative pressure to the secondary piping system 18 is started by the pressure transmission partition device 40 (time point at which it is switched from the positive pressure to the negative pressure), that the supplemental valve 72 is opened after a predetermined period of time (for example, 160 msec) after the time point at which the application of the negative pressure to the secondary piping system 18 is started (time point at which it is switched from the positive pressure to the negative pressure) (that is to say, 10 msec after the solenoid valve 12 is closed), and that the supplemental valve 72 is closed before the next switching (switching from the negative pressure to the positive pressure) (for example, 10 msec before the next switching).

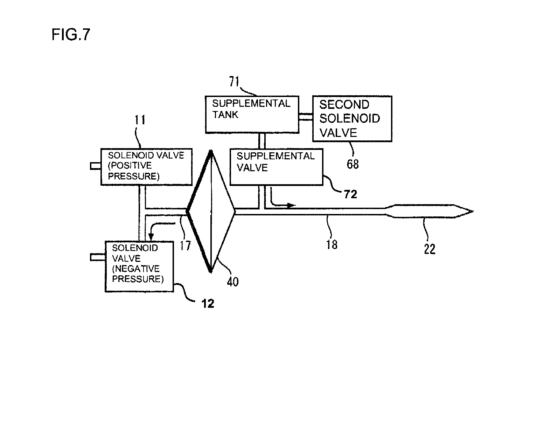

As illustrated in FIG. 7, when the supplemental valve 72 is opened in a state in which the solenoid valve 12 is opened and the negative pressure is applied to the secondary piping system 18, a part (or all) of the helium gas in the supplemental tank 71 is discharged into the secondary piping system 18 as indicated by an arrow in the drawing according to the difference between the pressure in the secondary piping system 18 and the pressure in the supplemental tank 71, and according to this, the pressure (reference pressure) in the secondary piping system 18 increases by an amount of the helium gas discharged by the supplemental tank 71.

Optimal time in relationship with the inflated state or a deflated state of the balloon 22 may be selected as timing at which the supplemental valve 72 is opened (predetermined period of time after the time point at which it is switched from the positive pressure or the negative pressure to the negative or the positive pressure), and this may set to a time point at which the balloon 22 is fully inflated or fully deflated, for example. The predetermined time (time point at which the balloon 22 is fully inflated or fully deflated) may be obtained, for example, by experimentally applying the positive pressure or the negative pressure to the balloon 22 using a water mock testing machine (for example, back pressure is set to 70 mmHg, gauge pressure) and measuring change in volume thereof.

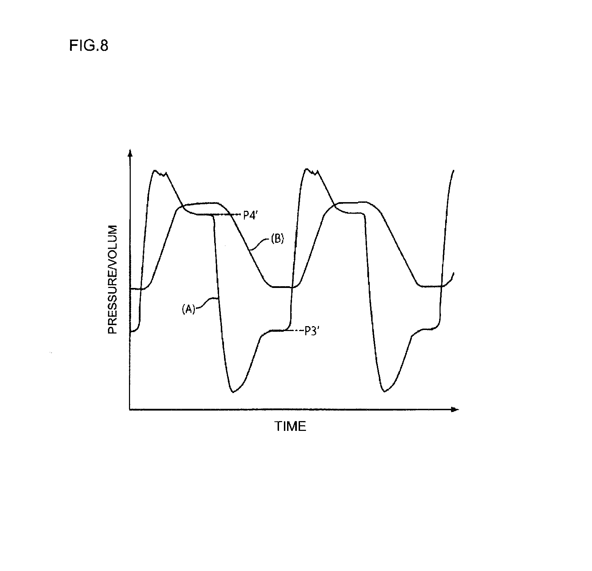

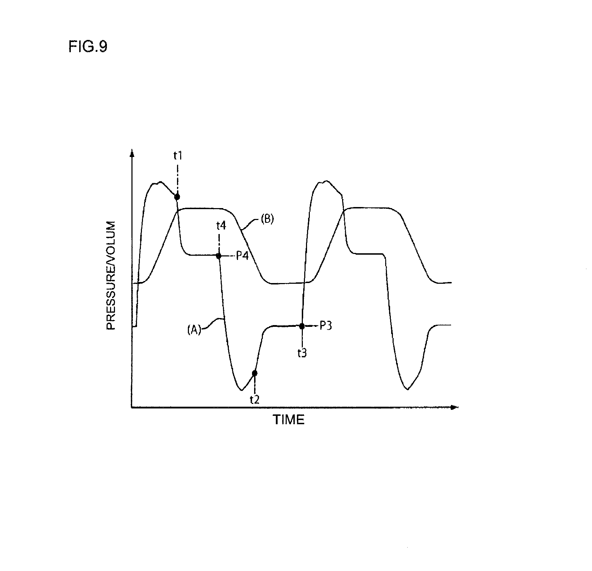

FIG. 8 is a graph illustrating the change in pressure in the secondary piping system 18 in a state in which the supplemental valve 72 is always closed and the supplemental device 70 is not allowed to operate, and FIG. 9 is a graph indicating the change in pressure in the secondary piping system 18 in a state in which the supplemental valve 72 is appropriately controlled to open and close as described above to allow the supplemental device 70 to operate. In FIGS. 8 and 9, an abscissa axis represents the time and a longitudinal axis represents the pressure or the volume, and a line indicated by a reference sign (A) represents the change in pressure in the secondary piping system 18 and a line indicated by a reference sign (B) represents the change in volume of the balloon 22.

In a case in which the supplemental device 70 is not allowed to operate, in FIG. 8, when the positive pressure is applied to the secondary piping system 18, the pressure in the secondary piping system 18 increases to overshoot plateau pressure (pressure when the balloon 22 is fully inflated) P4', then decreases to the plateau pressure P4' to be maintained substantially constant until the next switching (switching to the negative pressure), and by the switching to the negative pressure, the pressure in the secondary piping system 18 decreases to overshoot reference pressure (pressure when the balloon 22 is fully deflated) P3', then increases to the reference pressure P3' to be maintained substantially constant until the next switching (switching to the positive pressure), and they are sequentially repeated. As a result, the change in volume as indicated by the reference signal (A) in the drawing occurs, the inflation and the deflation of the balloon 22 in accordance with the heartbeat become possible, and it becomes possible to perform cardiac assist treatment.

In a case in which the supplemental device 70 is allowed to operate, in FIG. 9, when the positive pressure is applied to the secondary piping system 18, the pressure in the secondary piping system 18 increases to overshoot and then decreases, and at the time point at which the balloon 22 is fully inflated, the supplemental valve 72 is opened and the pressure in the secondary piping system 18 decreases to plateau pressure (pressure when the balloon 22 is fully inflated) P4 to be maintained substantially constant until the next switching (switching to the negative pressure), and by the switching to the negative pressure, the pressure in the secondary piping system 18 decreases to overshoot and then increases, and at the time point at which the balloon 22 is fully deflated, the supplemental valve 72 is opened and the pressure in the secondary piping system 18 increases to reference pressure (pressure when the balloon 22 is fully deflated) P3 to be maintained substantially constant until the next switching (switching to the positive pressure), and they are sequentially repeated. As a result, the change in volume as indicated by the reference signal (A) in the drawing occurs, the inflation and the deflation of the balloon 22 in accordance with the heartbeat becomes possible, and it becomes possible to perform the cardiac assist treatment.

As is clear from comparison between FIGS. 8 and 9, the plateau pressure decreases from P4' to P4 and the reference pressure increases from P3' to P3, so that it is possible to decrease difference between the plateau pressure and the reference pressure by allowing the supplemental device 70 to operate. According to this, it is possible to shorten time taken to reach the plateau pressure from the reference pressure at the time of the inflation, and on the other hand, shorten the time taken to reach the reference pressure from the plateau pressure at the time of the deflation, thereby improving the responsiveness.

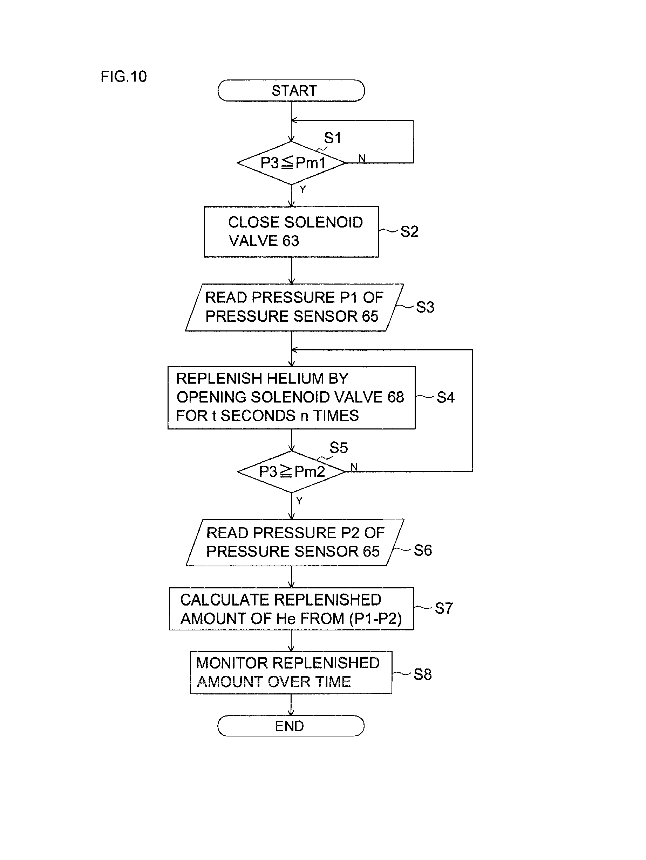

Next, operation control of the replenishment device 60 when replenishing the secondary piping system 18 with the helium gas or replacing (changing) the helium gas in the secondary piping system 18 with new helium gas is described with reference to a flowchart illustrated in FIG. 10. The control means 10 performs this control.

First, at step S1, the pressure in the secondary piping system 18 from the pressure sensor 15 illustrated in FIG. 3 is detected. At that time, in this embodiment, detected pressure by the pressure sensor 15 illustrated in FIG. 3 is detected at timing t3 at which the balloon is switched from the deflated state to the inflated state in FIG. 9 and it is judged whether the detected pressure (reference pressure) P3 is a predetermined value Pm1 or lower. If the detected pressure P3 is higher than the predetermined value Pm1, step S1 is repeated. The predetermined value Pm1 is set to, for example, 0 mmHg. A case in which the detected pressure P3 (FIG. 9) is the predetermined value Pm1 or lower is intended to mean a case in which the amount of the helium gas in the secondary piping system 18 decreases, and in this case, the procedure shifts to step S2 and subsequent steps to replenish the gas.

Meanwhile, in this embodiment, not the plateau pressure P4 (pressure when the balloon is inflated) illustrated in FIG. 9 but the pressure P3 in a state in which the balloon is deflated is used as the reference pressure for a following reason.

When the pressure on a balloon side is detected at timing t4 illustrated in FIG. 9 and the piping on the balloon side is replenished with the gas so as to maintain the pressure constant, there is an inherent risk of continuously using the balloon by making up deficiency in the helium gas as driver gas in the piping on the balloon side without noticing the change in balloon volume caused by an eventuality such as cyclic fatigue of the balloon, inadvertent pressurization (application of improper pressure, bend of patient's vessel), and catch by a projection in the patient's vessel at the time of insertion. Of course, an expected lifetime of the balloon deformed in this manner is shorter than that of the original one, so that this is not preferable for the patient. Also, when the inner pressure of the balloon becomes higher than a set upper limit value by increase in blood pressure associated with recovery of the patient, there is a risk that it is controlled to release the helium gas from the balloon and the balloon does not inflate in the worst case.

On the other hand, in this embodiment, the gas of a constant volume (constant moll number: chemical equivalent ratio) is put in the closed piping system 18 connected to the balloon 22 in the state in which the balloon 22 is deflated as illustrated in FIG. 9. Thereafter, reduction in gas, which decreases by permeation from the balloon 22 and the like is monitored necessarily in the state in which the balloon 22 is deflated.

Therefore, in this embodiment, it becomes possible to maintain the chemical equivalent of the gas corresponding to the volume of an optional drive piping system 18 (including tube and hose) and the balloon constant by eliminating an effect of the balloon 22 portion, which might be deformed by outer force, to the gas pressure. By controlling in this manner, it is possible to detect the change in volume of the balloon 22 by the eventuality such as bend of the balloon 22 by observing the plateau pressure (pressure in a state in which the balloon is inflated) P4 also. For example, when the plateau pressure P4 becomes higher than usual, it may be judged that the balloon 22 is bent. Also, when the plateau pressure P4 becomes lower than usual, it may be judged that the gas leaks by the eventuality other than the permeation.

Also, in this embodiment, when the blood pressure of the patient becomes higher than the plateau pressure P4, the volume of the balloon 22 is maintained substantially constant and the plateau pressure P4 remains to be substantially the same value as the blood pressure. Next, step S2 and subsequent steps illustrated in FIG. 10 are described.

When it is judged that replenishment of the gas is required at step S1, in this embodiment, the first solenoid valve 63 illustrated in FIG. 3 is closed at step S2. As a result, the communication between the primary helium gas tank 61 and the secondary helium gas tank 64 is blocked. Next, at step S3, pressure P1 of the tank pressure sensor 65 illustrated in FIG. 3 is read. Next, at step S4, in a state in which the supplemental valve 72 is closed, the second solenoid valve 68 is opened for t milliseconds n times to replenish the supplemental tank 71 with the helium gas from the gas tank 64. Although t is not especially limited, this is set to 8, for example. Although n is not especially limited, this is set to 1 to 10. The secondary piping system 18 is replenished with the helium gas in the supplemental tank 71 by next supplemental operation (opening of the supplemental valve 72).

Next, at step S5, the pressure P3 is read at the timing t3 illustrated in FIG. 9 by the pressure sensor 15 and it is judged whether the pressure P3 becomes a predetermined value Pm2 or higher. The value Pm2 is set to 10 mmHg, for example. Step S4 is repeated such that the pressure P3 becomes the predetermined value Pm2 or higher to replenish the gas.

At step S5, when the detected pressure P3 becomes the predetermined value Pm2 or higher, it may be judged that the gas is sufficiently replenished, so that the replenishment of the gas is completed, and a next step S6, detected pressure P2 by the pressure sensor 65 is read. The pressure P1 detected by the pressure sensor 65 is the pressure in the secondary helium tank 64 before the replenishment of the gas and the detected pressure P2 is the pressure in the secondary helium tank 64 after the gas replenishment is completed. Further, the communication between the primary helium tank 61 and the secondary helium tank 64 is blocked by the solenoid valve 63. Therefore, a replenished amount of the gas moved from the secondary helium tank 64 into the secondary piping system 18 (supplemental tank 71) is obtained from difference in pressure (P1-P2) and a volume V of the secondary helium gas tank 64 (measured in advance). The replenished amount is proportional to (P1-P2) × V.

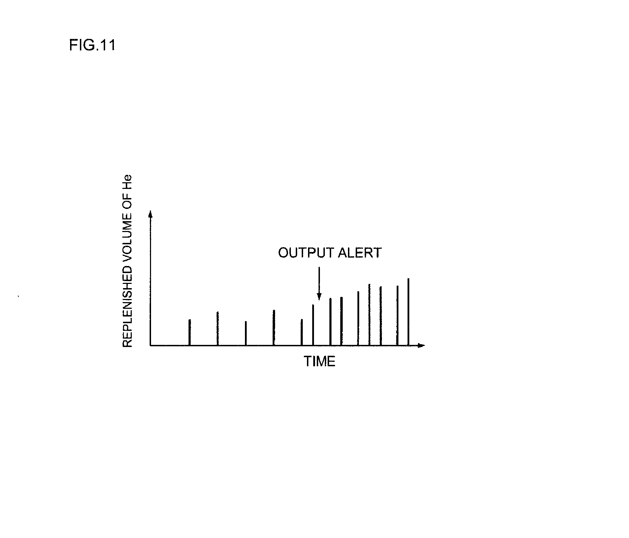

Next, at step S8, the replenished amount of the gas obtained in this manner is recorded over time. It is preferable to record on a semiconductor memory, a magnetic disk, an optical recording medium, and other recording media as recording means so as to be able to output on a screen or paper as needed. Then, while observing change over time in the recorded replenished amount of the gas, it is possible to judge that abnormal leakage of the gas occurs when an interval between each gas replenishment starts becoming shorter and the replenished amount of the gas starts increasing as illustrated in FIG. 11, thereby issuing an alert early. A central processing unit (CPU) and the like appropriately reads the change over time in the replenished amount of the gas recorded on the recording means, and when this judges that the replenished amount of the gas starts increasing, this may automatically issue the alert.

Also, when it is required to entirely change the helium gas in the secondary piping system 18 (full purge), the control means 10 performs following control. That is to say, in the state in which the supplemental valve 72 is closed, this opens the purge valve 73 to discharge the helium gas in the supplemental tank 71 to the vacuum tank 74 and closes the purge valve 73, thereafter opens the second solenoid valve 68 to supply the new helium gas into the supplemental tank 71 and closes the second solenoid valve 68.

The helium gas supplied into the supplemental tank 71 is supplied into the secondary piping system 18 by the next supplemental operation (opening of the supplemental valve 72). According to this, a part of the helium gas in the secondary piping system 18 is replaced with the new helium gas (half purge). Meanwhile, the supplemental operation is maintained also at that time. By repeating the operation an appropriate number of times, it is possible to entirely change the helium gas in the secondary piping system 18 (full purge). Conventionally, such gas change (full purge) is performed in a state in which the inflation and the deflation of the balloon 22 are temporarily stopped, it is not preferable to stop the inflation and the deflation of the balloon 22. On the other hand, in this embodiment, it is possible to entirely change (full purge) by replacing a part of the helium gas in the secondary piping system 18 (half purge) a plurality of times without stopping the inflation and the deflation of the balloon 22 by performing such control.

### (First Example)

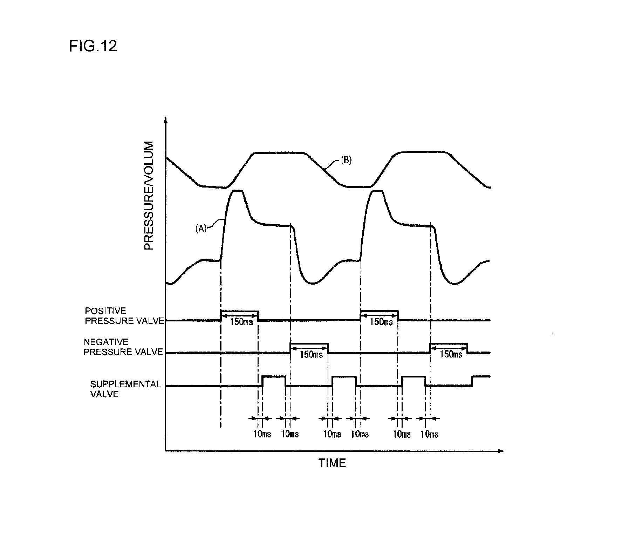

The helium gas was injected into the secondary piping system 18 such that the plateau pressure was 120 to 130 mmHg (gauge pressure), the opening/closing of the positive pressure valve (solenoid valve) 11, the negative pressure valve (solenoid valve) 12, and the supplemental valve 72 was set as illustrated in FIG. 12, that is to say, timing to open the supplemental valve 72 was set to 10 msec after the positive pressure valve 11 or the negative pressure valve 12 was closed, and summation of inflation time and deflation time was measured: this was 212 msec. Under the same condition, in a case in which the supplemental device 70 was not allowed to operate (supplemental valve 72 was always closed), the summation of the inflation time and the deflation time was 235 msec. Meanwhile, a volume of the supplemental tank 71, a diameter of the catheter, a length of a drive tube, and the volume of the balloon were set to 65 mL, 6 Fr (French: 1 Fr is nearly equal to 0.33 mm), 180 cm, and 35 mL, respectively. As seen from an experimental result, the summation of the inflation time and the deflation time can be decreased by approximately 10% by allowing the supplemental device 70 to operate, and it was confirmed that the responsiveness was improved.

### (Second Example)

Under the condition similar to that of the first example, the timing to open the supplemental valve 72 was set to 30 msec after the positive pressure valve 11 or the negative pressure valve 12 was closed and the summation of the inflation time and the deflation time was measured: this was 204 msec. Also, under the similar condition, the timing to open the supplemental valve 72 was set to 60 msec after the positive pressure valve 11 or the negative pressure valve 12 was closed and the summation of the inflation time and the deflation time was measured: this was 213 msec. From this result, it was proved that the timing to open the supplemental valve 72 was preferably set to approximately 30 msec after the positive pressure valve 11 or the negative pressure valve 12 was closed.

### (Third Example)

Under the condition similar to that of the first example, the timing to open the supplemental valve 72 was set to 30 msec after the positive pressure valve 11 or the negative pressure valve 12 was closed and the length of the drive tube was set to 230 cm, then the summation of the inflation time and the deflation time was measured: this was 213 msec. Since the summation of the inflation time and the deflation time was 204 msec when the length of the drive tube was 180 cm, it was confirmed that the drive tube is sufficiently practical even when the length thereof is increased by application of the present invention.

### (Fourth Example)

Under the condition similar to that of the third example, it was driven so as to be able to correspond to heart rates HR= 100 bpm, 120 bpm, 140 bpm, 160 bpm, and 180 bpm: the summations of the inflation time and deflation time were 210 msec, 215 msec, 221 msec, 230 msec, and 239 msec when the heart rates HR = 100 bpm, 120 bpm, 140 bpm, 160 bpm, and 180 bpm, respectively. From this result, it was confirmed that the sufficient responsiveness was obtained when the heart rate HR = 180 bpm or lower.

### (Second Embodiment)

Next, a second embodiment of the present invention is described. A medical inflation/deflation drive device of the second embodiment has a configuration similar to that of the above-described medical inflation/deflation drive device of the first embodiment, so that the description thereof is omitted.

In this embodiment, pressure in a secondary piping system 18 is made negative pressure, the pressure in the system is monitored by a pressure sensor 15 when the system is filled (replaced) with helium gas and the helium gas is enclosed therein until the pressure becomes the pressure determined by a volume of a balloon 22. For example, when a balloon catheter 20 having the volume of 40 cc is used, gas pressure when filling the secondary piping system 18 is set to +10 ± 5 mmHg (gauge pressure), and when the balloon catheter 20 having the volume of 30 cc is used, the gas pressure when filling the secondary piping system 18 is set to - 30±5 mmHg (gauge pressure) .

In this embodiment, by driving a pump 4a, pressure PT1 in a first pressure tank 2 is set to approximately 300 mmHg (gauge pressure) and by driving a pump 4b, pressure PT2 in a second pressure tank 3 is set to approximately -150 mmHg (gauge pressure). Then, pressure applied to an input port of a pressure transmission partition device 40 illustrated in FIG. 3 is switched to the pressure of the first pressure tank 2 and that of the second pressure tank 3 by alternately driving solenoid valves 11 and 12. Control means 10 controls switching timing in accordance with a heartbeat of a patient.

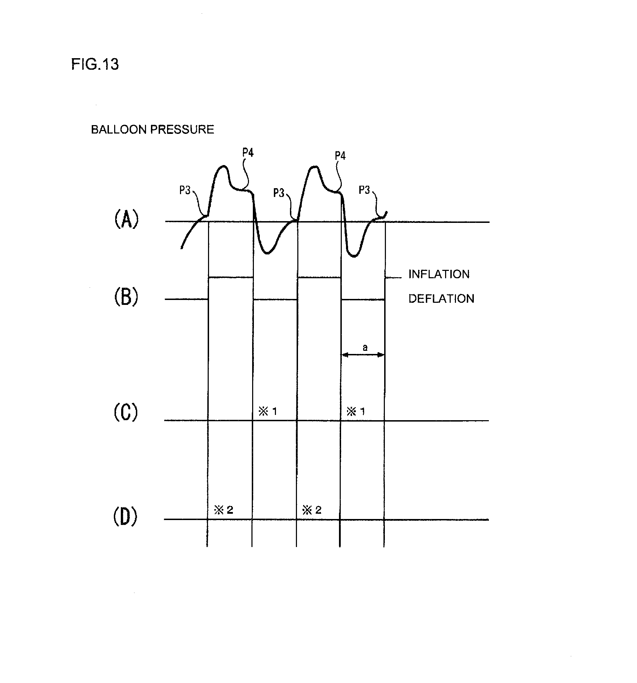

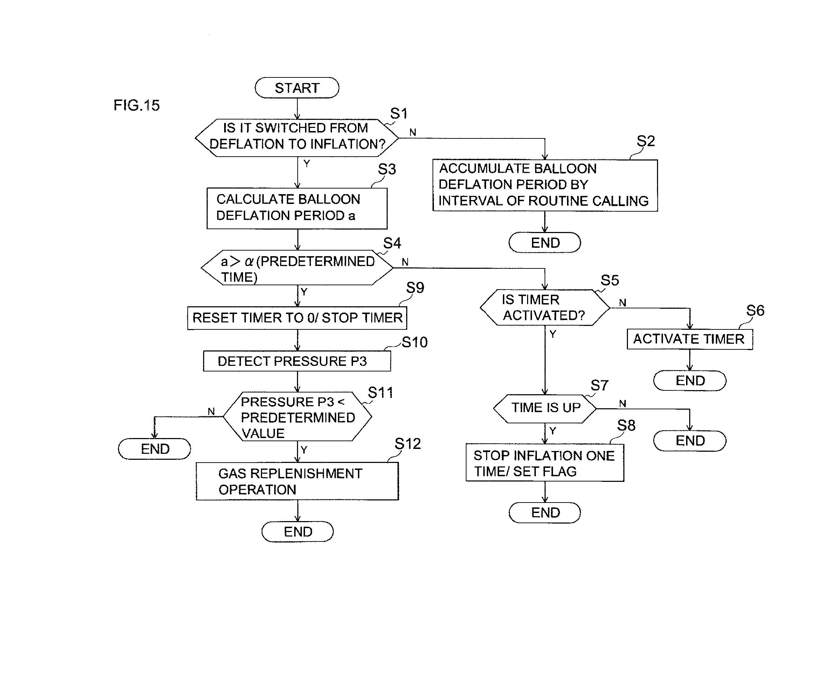

An example of detecting the pressure at a point P3 in FIG. 13 when the balloon is deflated is hereinafter described by operation illustrated in FIG. 15. In this routine, only a part of judging whether to replenish the gas is especially extracted. This routine is an interrupt routine called by a programmable timer and the like at regular intervals. The interval between calls is preferably set to approximately 1 to 20 milliseconds.

It is confirmed whether switching from deflation to inflation occurs at step S1. When this does not occur, a deflation period is accumulated at step S2 to finish the interrupt routine. When the switching from the deflation to the inflation is to occur at step S1, the procedure shifts to step S3 to calculate balloon deflation time a (refer to FIG. 13(B)). It is possible to calculate the balloon deflation time a by measuring switching time of the solenoid valves 11 and 12 illustrated in FIG. 3, for example. It is also possible to detect the pressure by the pressure sensor 15 illustrated in FIG. 3 to calculate based on change in pressure. Further, since the deflation time a is determined based on change in blood pressure or the heartbeat of the patient, it is also possible to calculate based on an output signal from a device, which detects the change in blood pressure or the heartbeat. The control means 10 illustrated in FIG. 3, which realizes step S3, corresponds to deflation/inflation time calculation means of the present invention.

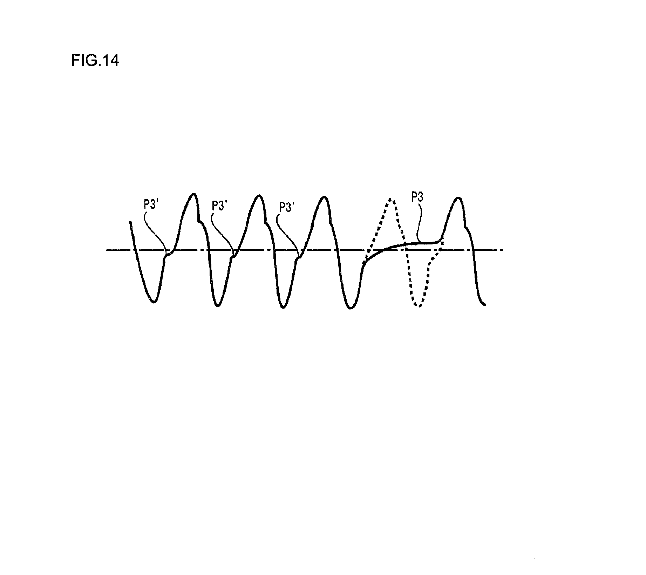

Next, at step S4 illustrated in FIG. 15, it is judged whether the deflation time a is shorter than predetermined time α. Although the predetermined time α is not especially limited, this is preferably set to 100 to 500 milliseconds, and is more preferably set to 150 to 300 milliseconds. When the balloon 22 is repeatedly inflated and deflated at intervals of the predetermined time or shorter, it is not possible to detect the pressure in a stable deflated state even when the pressure in the secondary piping system 18 is detected by the pressure sensor 15 at timing just before the balloon 22 is switched from the deflated state to the inflated state. For example, when the inflation and the deflation are repeated with such a short period, the change in pressure in the secondary piping system 18 is as illustrated in FIG. 14 and pressure P3' lower than the pressure P3 in the stable deflated state of normal operation of the balloon 22 (pulse is 50 to 100) is detected.

If the deflation time a is shorter than the predetermined time α at step S4, activation of a timer of software or hardware of which time is up after one to tens of minutes, and preferably three to ten minutes is confirmed (S5), and when this is not activated, this is activated (S6). When the timer is activated and further the time is up (S7), a flag, which stops the inflation one time, is set at S8 and inhibits operation of the solenoid valve for the inflation in the routine, which does not appear here, to maintain the deflated state. In general, a condition at the S4 is satisfied by the inhibition of one time and the procedure shifts to S9. At the S9, the timer is reset to 0 and is stopped. Also, the pressure P3 is detected at S10 and it is confirmed whether the pressure is predetermined pressure or lower at S11. If the pressure is the predetermined pressure or lower, gas replenishment operation is performed at S12. Meanwhile, when the gas replenishment operation is performed in another routine, it is possible to set a flag for this at the S12. Also, when a heart rate of the patient fluctuates and the condition at step S4 is occasionally satisfied, the timer is stopped and reset to 0, so that the procedure does not reach step S8 for inhibiting the inflation of the balloon. Of course, it is not applicable to a case in which the condition at step S4 is not established before the time of the timer is up. According to this, the inflation of the balloon is not unnecessarily inhibited to reduce assistance for the patient.

In the normal gas replenishment operation, the detected pressure by the pressure sensor 15 illustrated in FIG. 3 is detected at timing *2 illustrated in FIG. 13(D) (timing just before the balloon is switched from the deflated state to the inflated state in FIGS. 13(A) and (B)), a solenoid valve 68 is opened such that the detected pressure P3 (FIG. 13(A)) becomes a predetermined value, and the secondary piping system 18 is replenished with the gas through the supplemental tank 71. Although opening degree control of the solenoid valve 68 is not especially limited, this is the control to open the valve 68 at timing of 8 milliseconds × n times, for example. For example, n is set to 2 to 10.

At step S11, when the detected pressure P3 is lower than 0 mmHg, for example, the above-described gas replenishment operation is performed and the gas is replenished such that P3 is set to approximately 10 mmHg. In this embodiment, it is also possible to change reference pressure (threshold) to perform the gas replenishment according to the volume of the balloon 22. For example, it is possible to control such that P3 = +10±5 mmHg (gauge pressure) is satisfied when the volume is 40 cc and that P3 = -30±5 mmHg (gauge pressure) is satisfied when the volume is 30 cc. When the detected pressure P3 is lower than the values, the control means 10 controls to drive the solenoid valve 68 and replenish the secondary piping system 18 with the helium gas from a secondary helium gas tank 64 through the supplemental tank 71 such that the detected pressure P3 illustrated in FIG. 13(A) becomes the predetermined value.

In this manner, in the medical inflation/deflation drive device according to the second embodiment of the present invention, it is possible to replenish the secondary piping system 18 including the balloon 22 with an appropriate amount of gas even when the interval between each inflation/deflation of the balloon 22 is short. As a result, in this embodiment, it is possible to resolve various problems caused by too much gas injected into the secondary piping system 18 even when the patient has a rapid heart rate. Although the inflation of the balloon 22 is stopped for one beat or a few beats at that time in this embodiment, since it is for a short time, there is no effect on treatment by the balloon 22. Also, as compared to the drive device, which replaces the gas in an entire secondary piping system 18 at regular intervals, this embodiment is economic with a small amount of gas consumption.

Further, in the medical inflation/deflation drive device according to this embodiment, different from the Japanese Patent Application Laid-Open No. 05-10952, when the balloon catheter 20 is driven, the pressure P3 in the secondary piping system 18 is detected at timing just before the balloon 22 of the balloon catheter 20 is switched from the deflated state to the inflated state (FIG. 13 (D)) and the secondary piping system 18 is replenished with the gas such that the detected pressure P3 becomes the predetermined value. That is to say, although pressure (plateau pressure) P4 in a state in which the balloon 22 is inflated is detected as illustrated in FIG. 13(C) and this is controlled so as to be constant in the drive device disclosed in the above-described publication, the pressure P3 in a state in which the balloon 22 is deflated is detected and this is controlled so as to be the predetermined value in this embodiment. In other words, in this embodiment, the gas of a constant volume (constant moll number: chemical equivalent ratio) is injected into an enclosed piping system 18 connected to the balloon 22 in the state in which the balloon 22 is deflated. Thereafter, the gas amount decreased by permeation from the balloon 22 and the like is monitored necessarily in the state in which the balloon 22 is deflated.

Therefore, in this embodiment, it becomes possible to maintain a chemical equivalent of the gas according to the volume of an optional drive piping system 18 (including tube and hose) and the balloon constant by eliminating an effect of the balloon 22 portion, which might be deformed by outer force to the gas pressure. It is possible to detect change in volume of the balloon 22 by an eventuality such as bend of the balloon 22 by observing the plateau pressure (pressure in the state in which the balloon is inflated) P4 also by controlling in this manner. For example, when the plateau pressure P4 becomes higher than usual, it may be judged that the balloon 22 is bent. Also, when the plateau pressure P4 becomes lower than usual, it may be judged that the gas leaks by the eventuality other than the permeation.

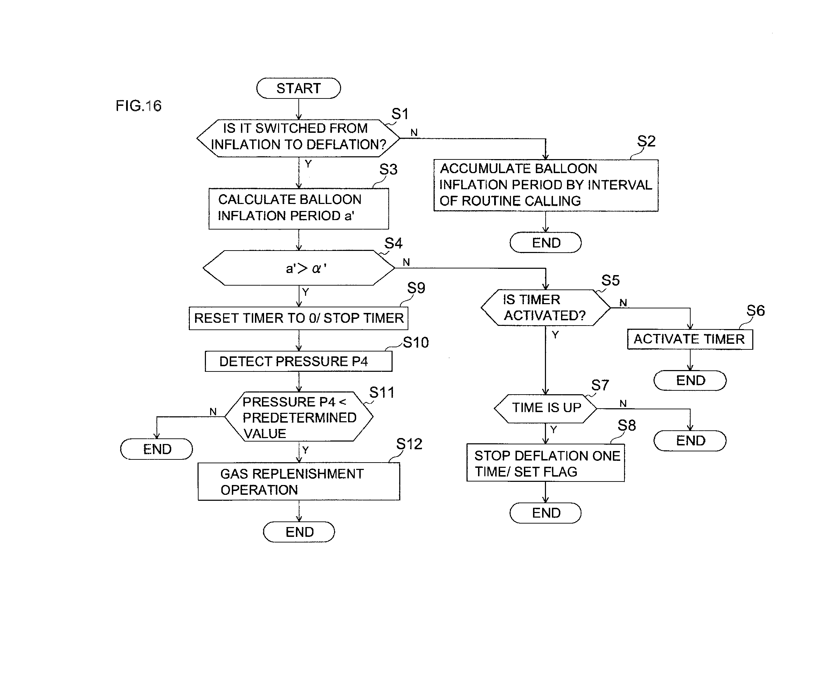

Of course, it is possible that the balloon inflation time is sufficiently longer than predetermined time, the stable pressure P4 is detected, and this embodiment is modified such that the pressure value is maintained at a predetermined value even in a state in which such defects are included. The routine at that time is illustrated in FIG. 16. Detailed description thereof is similar to that in FIG. 15 except "inflation" is changed to "deflation" and "deflation" is changed to "inflation".

### (Third Embodiment)

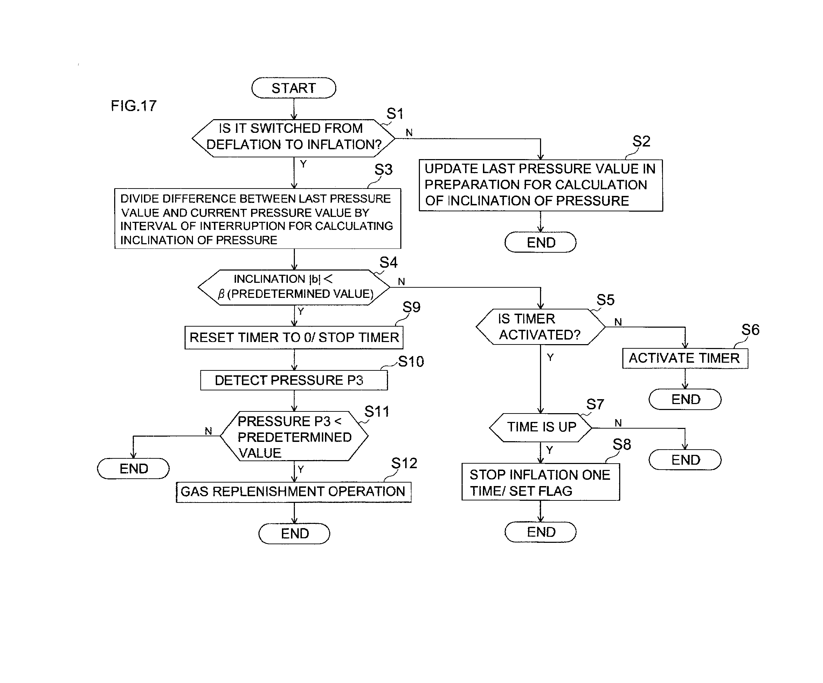

Next, a medical inflation/deflation drive device according to a third embodiment of the present invention is described. The medical inflation/deflation drive device of this embodiment has the same configuration as that of the medical inflation/deflation drive device of the second embodiment except a function of control means 10. Therefore, the description of a part common to the second embodiment is omitted and only a different part is described with reference to FIG. 17.

At step S2 in FIG. 17, a pressure value used as a last pressure value is updated in order to calculate inclination of pressure. Next, when it is switched from deflation to inflation and the inclination of the pressure is calculated at step S3, inclination b of the pressure is obtained by obtaining difference between the last pressure value and a current pressure value and dividing the difference by a time interval of measurement of the pressure value. An absolute value of the inclination b is compared with a predetermined value β at step S4. Although the predetermined value β is not especially limited, this is set to 0 to 100 mmHg/sec and is preferably set to 0 to 50 mmHg/sec. Operation of a part other than this is similar to that of the second embodiment.

In this manner, in the medical inflation/deflation drive device according to the third embodiment of the present invention, when the inclination b of change in pressure calculated at step S3 is smaller than the predetermined value β and is stable (case in FIG. 13(A)), pressure in the secondary piping system 18 is detected and gas replenishment operation is performed based on the pressure. Also, when the inclination b of the change in pressure is larger than the predetermined value β (case in FIG. 13(A)), at step S5, the gas replenishment is not performed for a while and it is waited until the inclination b of the change in pressure becomes smaller than the predetermined value β. When the inclination b of the change in pressure does not become smaller than the predetermined value β within the predetermined time, at step S8, the inflation is stopped one or more times to create a condition that this becomes smaller. Pressure P3 in the secondary piping system 18 is detected in this state and the gas replenishment operation is performed based on the pressure P3.

Therefore, in this embodiment, it is possible to replenish the secondary piping system 18 including the balloon 22 with an appropriate amount of gas. As a result, in this embodiment, too much gas is not injected into the secondary piping system 18 even when a heartbeat of a patient becomes rapid and various problems caused by this may be resolved. Also, in this embodiment, since the inflation and deflation of the balloon 22 do not basically stop at that time, there is no effect on treatment by the balloon. Also, as compared to the drive device, which replaces the gas in an entire piping system at regular intervals, this embodiment is economic with a small amount of gas consumption.

Further, just the same as the second embodiment, it is also possible to deform so as to make plateau pressure in an inflated state of the balloon constant.

Meanwhile, the present invention is not limited to the above-described embodiment and may be modified variously within the scope of the present invention.

For example, although two pumps 4a and 4b are used as primary side pressure generation means in the above-described embodiment, in the present invention, it is also possible to use a single pump and connect a first pressure tank 2 as a positive pressure tank to a positive pressure output port thereof and connect a second pressure tank 3 as a negative pressure tank to a negative pressure output port of the pump. In this case, it is possible to reduce the number of pumps, thereby contributing to a light weight and energy saving of a device. Also, not only a diaphragm pump but also a linear piston pump, a rotary vane pump, a piston pump, a compressor and the like may be used as the pump.

Although two solenoid valves 11 and 12 are used as pressure switch means in the above-described embodiment, the present invention is not limited to this and it is also possible to switch the pressure applied to an input port of a pressure transmission partition device 40 by using a single three-directional changeover valve.

Further, a type of the gas in a primary piping system 17 is not limited to air and this may be another fluid. Also, the type of the gas in the secondary piping system 18 is not limited to helium gas and this may be another fluid.

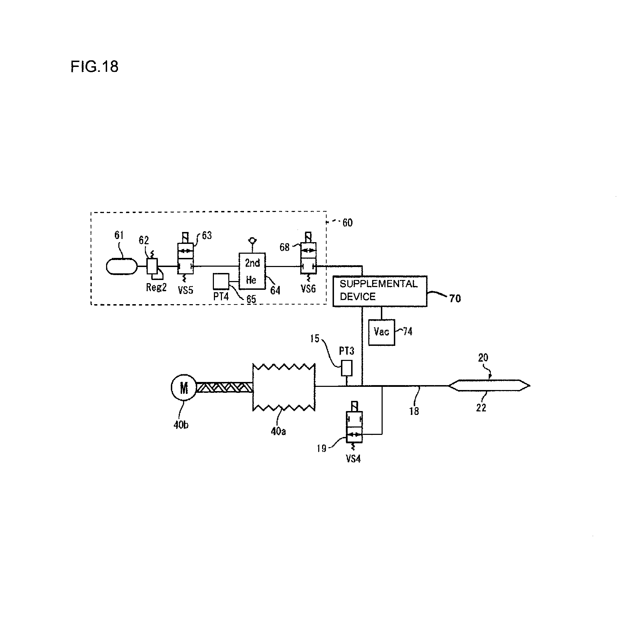

Further, it is also possible to use pressure generation means, which allows a predetermined volume of gas to directly reciprocate in the secondary piping system 18 as illustrated in FIG. 18 without using the primary piping system 17 and the pressure transmission partition device 40. The pressure means is composed of, for example, a bellows 40a and drive means, which drives the bellows 40a to expand and contract in an axial direction (for example, motor 40b), and this allows the inside or the outside of the bellows 40a to directly communicate with the inside of the secondary piping system 18. By allowing the bellows 40a to reciprocate in the axial direction by means of the motor 40b and the like, the gas is allowed to directly reciprocate in the secondary piping system 18 at predetermined timing to inflate and deflate the balloon 22. Another configuration is similar to that in FIG. 3.

Although a balloon catheter is used as a driven device in the above-described embodiment, the drive device according to the present invention may also be used for driving another medical device as long as this is the medical device, which is repeatedly inflated and deflated.

### {Reference Signs List}

- 2: first pressure tank
- 3: second pressure tank
- 4a, 4b: pump
- 5, 6: pressure sensor
- 10: control means
- 11, 12, 16, 19: solenoid valve
- 15: pressure sensor (piping system pressure detection means)
- 17: primary piping system
- 18: secondary piping system
- 20: balloon catheter
- 22: balloon
- 40: pressure transmission partition wall
- 60: replenishment device (gas replenishment means)
- 61: primary helium gas tank (primary gas tank)
- 63: first solenoid valve (first valve means)
- 64: secondary helium gas tank (secondary gas tank)
- 65: pressure sensor (tank pressure detection means)
- 68: second solenoid valve (second valve means)
- 70: supplemental device
- 71: supplemental tank (gas tank)
- 72: supplemental valve (valve means)
- 73: purge valve
- 74: vacuum tank

## Claims

1. A medical inflation/deflation drive device, comprising:
pressure generation means, which, in order to repeat inflation and deflation of a driven device, alternatively applies positive pressure and negative pressure to a piping system communicating with the driven device;
a gas tank, which sucks a part of gas in the piping system or discharges a part of the gas in the gas tank to the piping system according to difference between pressure in the piping system and the pressure in the gas tank;
valve means, which selectively opens and closes communication between the gas tank and the piping system; and
control means, which controls in such a manner that the valve means is opened after a predetermined period of time after a point of time at which application of the pressure by the pressure generation means is switched from the application of the positive pressure or the negative pressure to the application of the negative pressure or the positive pressure, and that the valve means is closed before next switching.

2. A medical inflation/deflation drive device; comprising:
pressure generation means, which, in order to repeat inflation and deflation of a driven device, alternately applies positive pressure and negative pressure to a piping system communicating with the driven device;
a gas tank, which sucks a part of gas in the piping system or discharges a part of the gas in the gas tank to the piping system according to difference between pressure in the piping system and the pressure in the gas tank;
valve means, which selectively opens and closes communication between the gas tank and the piping system;
gas replenishment means, which replenishes the piping system with the gas through the gas tank;
gas discharge means, which discharges the gas in the piping system through the gas tank; and
control means, which controls in such a manner that the valve means is opened after application of the pressure by the pressure generation means is switched from the application of the positive pressure or the negative pressure to the application of the negative pressure or the positive pressure and that the valve means is closed before next switching, and, in a state in which the valve means is closed, controls the gas discharge means to discharge at least a part of the gas in the gas tank and controls the gas replenishment means to replenish the gas tank with the gas.

3. The medical inflation/deflation drive device according to claim 2, further comprising:
piping system pressure detection means, which detects the pressure in the piping system; and
gas replenishment means, which replenishes the piping system with the gas through the gas tank, wherein
the gas replenishment means includes:
a primary gas tank in which the gas with which the gas tank is replenished is enclosed;
first valve means, which may be opened and closed, connected to an output side of the primary gas tank;
a secondary gas tank, which communicates with the output side of the primary gas tank by opening and closing of the first valve means;
tank pressure detection means, which detects the pressure in the secondary gas tank; and
second valve means connected to an output side of the secondary gas tank, which controls replenishment of the gas tank with the gas from the secondary gas tank by the opening and closing of the valve, and wherein
when the pressure detected by the piping system pressure detection means at timing at which the driven device is switched from a deflated state to an inflated state becomes a predetermined value or lower, the control means closes the first valve means and opens the second valve means before opening the valve means to replenish the gas tank with the gas from the secondary gas tank and calculates a replenished amount of the gas to the piping system based on change in pressure in the secondary gas tank detected by the tank pressure detection means before and after the replenishment of the gas.

4. The medical inflation/deflation drive device according to claim 2, further comprising:
deflation time calculation means, which calculates time during which the driven device is deflated;
inflation stop means, which stops inflation of the driven device one or more times continuously when deflation time calculated by the deflation time calculation means is predetermined time or shorter to make the deflation time the predetermined time or longer;
pressure detection means capable of detecting the pressure in the piping system at timing just before it is switched to next inflation after the inflation is stopped one or more times by the inflation stop means; and
gas replenishment means, which replenishes the piping system with the gas through the gas tank such that the pressure detected by the pressure detection means becomes a predetermined value.

5. The medical inflation/deflation drive device according to claim 2, further comprising:
inflation time calculation means, which calculates time during which the driven device is inflated;
inflation continuation means, which, when inflation time calculated by the inflation time calculation means is a predetermined time or shorter, continues the inflation of the driven device for predetermined time or longer;
pressure detection means capable of detecting the pressure in the piping system at timing just before it is switched to next deflation after the inflation continuation means continues the inflation for the predetermined time or longer; and
gas replenishment means, which replenishes the piping system with the gas through the gas tank such that the pressure detected by the pressure detection means becomes a predetermined value.

6. The medical inflation/deflation drive device according to claim 2, further comprising:
pressure detection means, which detects inner pressure of the piping system;
pressure change calculation means, which calculates inclination of change in pressure of the inner pressure of the piping system by the pressure detection means at timing just before the driven device is switched from a deflated state or an inflated state to the inflated state or the deflated state;
gas replenishment stop means, which stops gas replenishment operation to the piping system when an absolute value of the inclination of the change in pressure calculated by the pressure change calculation means is larger than a predetermined value; and
gas replenishment means, which replenishes the piping system with the gas through the gas tank such that the inner pressure of the piping system detected by the pressure detection means becomes a predetermined value at the timing just before the driven device is switched from the deflated or the inflated state to the inflated state or the deflated state when the absolute value of the inclination of the change in pressure calculated by the pressure change calculation means is equal to or lower than the predetermined value.
